# EUROPEAN PATENT APPLICATION

(11) **EP 4 252 733 A1**
(43) Date of publication of application: **04.10.2023**
(21) Application number: 21898115.7
(22) Date of filing: 26.11.2021
(51) Int. Cl.: A61H 1/02, G09B 7/02, G09B 19/00, A61B 10/00

(54) **EVALUATION DEVICE, METHOD, AND PROGRAM FOR EVALUATING ABILITY TO IDENTIFY OBJECT**

(30) Priority: 26.11.2020 JP 2020196171
(71) Applicant: Sumitomo Pharma Co., Ltd., Osaka 541-0045 (JP)
(72) Inventor: OCHIAI Yasushi, Tokyo 103-6012 (JP); KASAI Kazuki, Tokyo 158-0094 (JP)
(74) Representative: Zimmermann & Partner Patentanwälte mbB
(86) International application number: PCT/JP2021/043460
(87) International publication number: WO 2022/114143

(57) **Abstract**

Provided is an evaluation device and the like that can objectively evaluate an ability to identify an object while further reducing a burden on a test subject. The present invention is an evaluation device for evaluating a test subject's ability to identify an object, the evaluation device including: an input unit that receives input of the test subject; a display control unit that displays, on a display device, a test screen including a target object that the test subject should select and a non-target object that the test subject should not select; an input processing unit that determines whether or not a target object is selected on the basis of input of the test subject received by the input unit; and an evaluation unit that evaluates a temporal lobe function related to a visual recognition ability of the test subject on the basis of a response time required for the test subject to input when the input processing unit determines that the target object is selected.

## Description

### Technical Field

The present invention relates to an evaluation device, a method, and a program, and particularly relates to an evaluation device, a method, and a program for evaluating a test subject's ability to identify an object.

### Background Art

It is known that a processing path of visual information of a brain is mainly divided into two paths of a dorsal side (parietal lobe) path and a ventral side (temporal lobe) path, the parietal lobe path is in charge of spatial information such as movement and the three-dimensional structure of a space, and the ventral side (temporal lobe) path is in charge of object identification based on color and shape. Regarding the visual recognition characteristics, it is known that there are those whose parietal lobe site is more activated and those whose temporal lobe site is more activated when visually recognizing an object, a person, or the like. For example, when memorizing a person's face, the former classifies features of the face and memorizes the features as an impression, whereas the latter memorizes the face itself as an image. For example, Non-Patent Document 1 discloses a relationship between brain activities and reaction time in a mental rotation task, and discloses that the brains of the test subjects, divided into a group with a fast reaction time and a group with a slow reaction time, are activated at different sites between the two groups. Since brain activities enables to more quantitatively grasp a disease state as compared with a diagnostic index mainly based on medical interview by a medical doctor, application of brain activities to state evaluation of mental diseases and the like is also studied.

### Citation List

### Patent Document

Patent Document 1: Japanese Patent Application Laid-Open No. 2001-79050

### Non-Patent Document

Non-Patent Document 1: Fumi Kusumoto, Ryota Imai, Takayuki Kodama, and Shu Morioka, "Relation between Brain Activity and Reaction Time in a Mental Rotation Task", Physical Therapy Science, Society of Physical Therapy Science, 2014, Vol. 29, No. 4, p. 479-483
Non-Patent Document 2: Alexander Provost, Blake Johnson, Frini Karayanidis, Scott D. Brown, Andrew Heathcote, "Two Routes to Expertise in Mental Rotation", Cognitive Science 37 (2013) 1321-1342

### Summary of Invention

### Technical Problem

The inventors of the present application consider that enabling objective evaluation through quantification of the temporal lobe function when visually recognizing an object, a person, or the like is extremely useful in order to realize a society where value is put not on the ability to excel in society but personality. Such techniques and technical ideas have not been considered so far. On the other hand, measurement such as brain waves and fMRI for quantitatively grasping characteristics of the brain requires a large device, and a burden on the test subject has been large because the measurement takes a long time and physical restraint is also involved.

The present invention has been made to solve such a problem, and a main object is to provide an evaluation device and the like that can objectively evaluate an ability to identify an object while further reducing a burden on a test subject.

### Solution to Problem

An evaluation device of one embodiment of the present invention is
an evaluation device for evaluating a test subject's ability to identify an object, the evaluation device including:
an input unit that receives input of the test subject;
a display control unit that displays, on a display device, a test screen including a target object that the test subject should select and a non-target object that the test subject should not select;
an input processing unit that determines whether or not a target object is selected on the basis of input of the test subject received by the input unit; and
an evaluation unit that evaluates the test subject's ability to identify an object on the basis of a response time required for the test subject to input when the input processing unit determines that the target object is selected.

In one embodiment of the present invention, when the input of the test subject received by the input unit is an input corresponding to selection of a target object or a non-target object, the input processing unit receives the input of the test subject as an object selection input, and
when the input processing unit determines that the target object is selected, the evaluation unit determines, as a response time, a time from when the display control unit displays a test screen to when the input processing unit receives an object selection input or a time from when the input processing unit receives an object selection input immediately before the object selection input to when the input processing unit receives the object selection input, and determines an index of the ability to identify an object on the basis of the determined response time to evaluate the test subject's ability to identify an object.

In one embodiment of the present invention, the display control unit displays, on a display device, a test screen including a plurality of target objects, and
the evaluation unit evaluates the test subject's ability to identify an object by determining a response time in each case where the input processing unit determines that a target object is selected, and determining an index of the ability to identify an object on the basis of the determined response time.

In one embodiment of the present invention, the display control unit displays a test screen including a non-target object that has a predetermined similarity to the target object in figure feature, color, or pattern target but is different from the target object in figure feature, color, or pattern target.

In one embodiment of the present invention, the display control unit displays, on the display device, a test screen including a target object and a non-target object rotated at a random angle around a predetermined position in each of the objects.

In one embodiment of the present invention, the display control unit displays, on the display device, a test screen including only one target object, and the target object is a UI object selectable by a test subject including a message for the test subject.

In one embodiment of the present invention, the display control unit sequentially displays different test screens on the display device while satisfying a predetermined condition, and
the evaluation unit evaluates the test subject's ability to identify an object by determining a response time on each of the test screens, and determining an index of the ability to identify an object on the basis of the determined response time.

In one embodiment of the present invention, the input processing unit determines whether or not a target object or a non-target object is selected on the basis of an input of the test subject received by the input unit, and
the evaluation unit outputs information indicative of not evaluating the ability to identify an object or not being able to evaluate the ability to identify an object in a case where a ratio between the number of times the input processing unit determines that the target object is selected and the number of times the input processing unit determines that the non-target object is selected falls within a predetermined range.

One embodiment of the present invention further includes a visual line detection unit that detects a visual line of the test subject,
in which the input processing unit does not determine that a target object is selected in a case where the detected visual line is not present in a predetermined region including an object in a test screen displayed by the display control unit.

In one embodiment of the present invention, the evaluation unit evaluates the test subject's ability to identify an object on the basis of a ratio between the number of times the input processing unit determines that the target object is selected and the number of times the input processing unit determines that the non-target object is selected, and a response time required for the test subject to input when the input processing unit determines that the target object is selected.

A method of one embodiment of the present invention is a method for evaluating a test subject's ability to identify an object, the method including:
a step of displaying, on a display device, a test screen including a target object that the test subject should select and a non-target object that the test subject should not select;
a step of determining whether or not a target object is selected on the basis of input of the test subject having been received; and
a step of evaluating the ability to identify an object on a basis of a response time required for the test subject to input when the target object is determined to have been selected.

A program of one embodiment of the present invention is characterized by causing a computer to execute each of the steps of the above method.

### Advantageous Effects of Invention

According to the present invention, it is possible to objectively evaluate an ability to identify an object while further reducing a burden on a test subject.

### Brief Description of Drawings

FIG. 1 is a hardware configuration diagram of an evaluation device according to one embodiment of the present invention.
FIG. 2 is a functional block diagram of the evaluation device according to one embodiment of the present invention.
Fig. 3 is a view illustrating an example of a test screen displayed by a display control unit.
Fig. 4 is a view illustrating an example of a mode selection screen displayed by the display control unit.
Fig. 5 is a view illustrating an example of a start screen displayed by the display control unit.
FIG. 6 is a view illustrating an example of an evaluation screen displayed by the display control unit.
Fig. 7 is a view illustrating an example of a flowchart explaining information processing executed in the evaluation device of one embodiment of the present invention.
Fig. 8 is a view illustrating an example of a flowchart explaining the information processing executed in the evaluation device of one embodiment of the present invention.
Fig. 9 is a view illustrating an example of a test screen displayed by the display control unit.

### Description of Embodiments

Hereinafter, an evaluation device 1 of the embodiment of the present invention will be described with reference to the drawings. In the present description, for convenience of description, detailed description more than necessary may be omitted. For example, detailed description of already well-known matters and redundant description of substantially the same configuration may be omitted.

The evaluation device 1 is a device that presents a test screen to a test subject to allow the test subject to visually recognize an object, and evaluates the test subject's ability to identify an object. For example, the evaluation of the ability to identify (recognize) an object is to evaluate whether a parietal lobe site is more activated or a temporal lobe site is more activated when visually recognizing an object such as an object or a person. The parietal lobe site being more activated indicates better the ability to grasp spatial information, and the temporal lobe site being more activated indicates a better eidetic memory ability. For example, the ability to identify an object can be evaluated by determining an index of the ability to identify an object. In the present embodiment, evaluating the ability to identify an object means evaluating the temporal lobe function related to the visual recognition ability of the test subject, and means evaluating the degree to which the temporal lobe is activated or used when the test subject visually recognizes the object, for example. In the present embodiment, the evaluation device 1 evaluates the temporal lobe function by determining an index of the temporal lobe function related to the visual recognition ability of the test subject. Determining an index of the temporal lobe function includes calculating the index. The object is a virtual object displayed in the display device. The test subject means someone who is evaluated after taking a test performed (presented) by the evaluation device 1, and can mean an evaluation target person or a measurement target person.

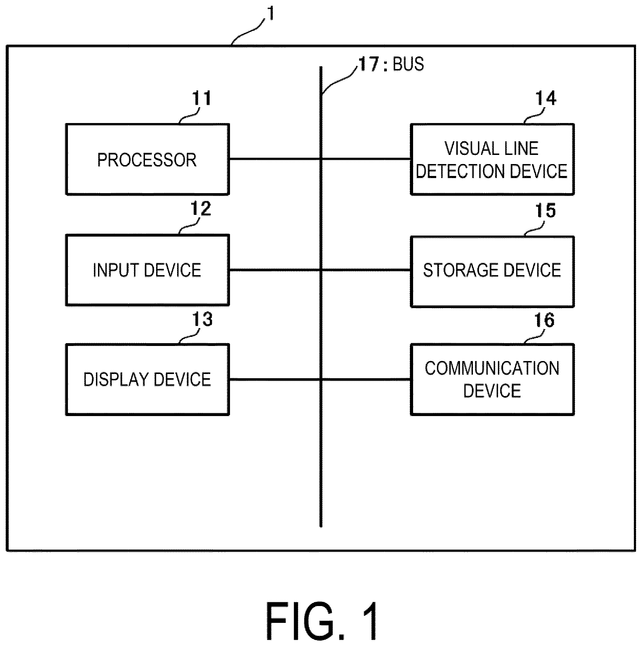

FIG. 1 is a block diagram illustrating a hardware configuration of the evaluation device 1 of one embodiment of the present invention. The evaluation device 1 includes a processor 11, an input device 12, a display device 13, a visual line detection device 14, a storage device 15, and a communication device 16. These constituent devices are connected by a bus 17. An interface is interposed between the bus 17 and each constituent device as necessary. The evaluation device 1 can be a computer, a tablet terminal, a smartphone, or the like. The evaluation device 1 may include one device or may include a plurality of devices.

The processor 11 controls the entire operation of the evaluation device 1. For example, the processor 11 is a CPU. The processor 11 executes various processing by reading and executing programs and data stored in the storage device 15. The processor 11 may include a plurality of processors.

The input device 12 is a user interface that receives input from the user to the evaluation device 1, and is, for example, a touchscreen, a touch pad, a mouse, a keyboard, or a sensor. The display device 13 is a display that displays an application screen or the like to the user of the evaluation device 1 under the control of the processor 11. In the present embodiment, the input device 12 is a touchscreen 18 and has a structure integrated with the display device 13 (display).

The visual line detection device 14 is a known eye tracking device or visual line measurement device. The visual line detection device 14 includes an imaging device for detecting a visual line. In one example, the visual line detection device 14 includes an infrared camera and an infrared LED. The visual line detection device 14 may be a visual line detection device module or the like, and may be built in the evaluation device 1. Alternatively, the visual line detection device 14 may include a plurality of devices.

The storage device 15 includes a main storage device and an auxiliary storage device. The main storage device is, for example, a semiconductor memory such as a RAM. The RAM is a volatile storage medium capable of reading and writing information at a high speed, and is used as a storage area and a work area when the processor 11 processes information. The main storage device may include a ROM, which is a read-only nonvolatile storage medium. The auxiliary storage device stores various programs and data used by the processor 11 when executing those programs. The auxiliary storage device may be any nonvolatile storage or nonvolatile memory as long as it can store information, and may be removable.

The storage device 15 stores an evaluation program for evaluating the temporal lobe function related to the visual recognition ability of the test subject, image data of an object referred to by the program, and the like.

The communication device 16 exchanges data with another computer such as a user terminal or a server via a network, and is, for example, a wireless LAN module. The communication device 16 can be another wireless communication device or module such as a Bluetooth (registered trademark) module, or can be a wired communication device or module such as an Ethernet (registered trademark) module or a USB interface.

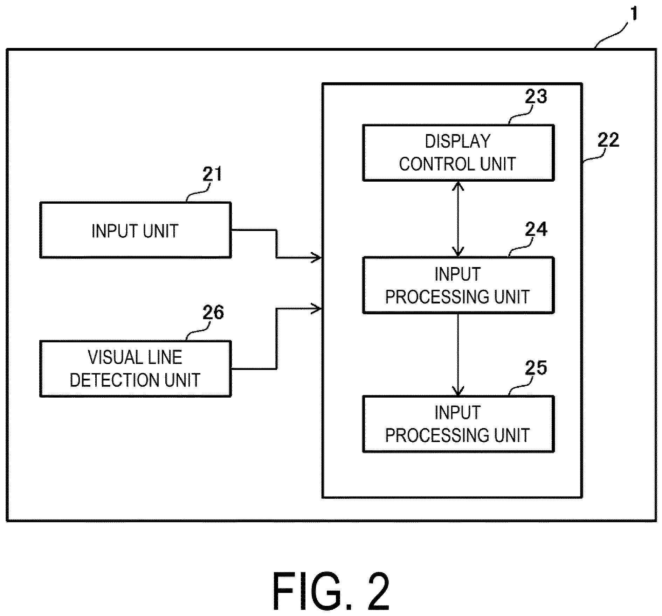

FIG. 2 is a functional block diagram of the evaluation device 1 of one embodiment of the present invention. The evaluation device 1 includes an input unit 21, a control unit 22, and a visual line detection unit 26. The control unit 22 includes a display control unit 23, an input processing unit 24, and an evaluation unit 25. In the present embodiment, these functions are implemented by the processor 11 executing the program stored in the storage device 15. As described above, since various functions are implemented by reading a program, some or all of the functions of one functional block may be included in another functional block. However, these functions may be implemented also by hardware by configuring an electronic circuit or the like for implementing some or all of the functions. For example, the functions of the display control unit 23 and the input processing unit 24 may be implemented by one functional block, may be implemented by more functional blocks, or some of the functions may be implemented by another functional block.

The input unit 21 receives input of the test subject. The input unit 21 receives an input of the test subject for selecting an object 40 on a test screen 30 displayed by the display control unit 23, for example. In the present embodiment, the input unit 21 is configured using the touchscreen 18, and acquires a touch position by receiving a touch input of the user to the touchscreen 18. As described above, the input unit 21 is a function generally included in a tablet terminal or a smartphone. In one example, the input unit 21 stores the acquired touch position in a predetermined memory area in the storage device 15 and passes it to the input processing unit 24. At this clock time, the input unit 21 acquires a touch position and acquires clock time (information regarding clock time) when the input unit 21 acquires the touch position, stores the clock time in a predetermined memory area in the storage device 15 in association with the touch position, and passes it to the input processing unit 24. The clock time when the input unit 21 acquires the touch position can mean the clock time when the input unit 21 receives the input. The input unit 21 and the control unit 22 can acquire information regarding clock time from, for example, an OS or the like.

The control unit 22 performs a test on the test subject via the test screen 30 and evaluates the temporal lobe function related to the visual recognition ability of the test subject.

The display control unit 23 displays, on the display device 13, the test screen 30 including a plurality of the objects 40. The plurality of objects 40 include a target object 41 that the test subject should select and a non-target object 42 that the test subject should not select. The target object 41 and the non-target object 42 are different from each other in at least one of a figure feature, a color, and a pattern. The display control unit 23 displays a predetermined number of the target objects 41 and the non-target objects 42 set in advance at predetermined respective positions set in advance according to the test screen 30. In the present embodiment, each of the objects 40 displayed by the display control unit 23 is a two-dimensional object having no information in the depth direction of the test screen 30. In the present embodiment, each of the objects 40 displayed by the display control unit 23 is a drawing such as an illustration. However, each of the objects 40 displayed by the display control unit 23 can be a three-dimensional object or an image instead of a drawing.

In the present embodiment, the display control unit 23 displays the test screens 30 of various patterns on the display device 13 according to the content of the evaluation program, for example. In one example, the test screen 30 includes a reference object 43 indicating the target object 41 that the test subject should select. In one example, the test screen 30 includes a user interface (UI) object 44. The UI object 44 is an object that includes a message for the test subject and can be selected by the test subject.

In one example, the display control unit 23 displays, on the display device 13, the test screen 30 including the target object 41 and the non-target object 42 having a predetermined similarity to the target object 41 in shape (e.g. figure shape), color, or pattern. In this case, in one example, the non-target object 42 is an object that has a predetermined similarity to the target object 41 in at least one of the figure feature, the color, and the pattern. Alternatively, in one example, the non-target object 42 is an object that is the same as the target object 41 in at least one of the figure feature, the color, and the pattern and that is different from the target object 41 in at least one of the figure feature, the color, and the pattern. The figure feature means a figure feature of at least a part of the object. For example, the target object 41 and the non-target object 42 are objects that are possibly give a similar impression when viewed at a glance.

In one example, the display control unit 23 displays, on the display device 13, the test screen 30 including the target object 41 and the non-target object 42 rotated at random angles around predetermined positions in the respective objects 40. For example, each of the target object 41 and the non-target object 42 includes information regarding a preset orientation. The display control unit 23 displays the test screen 30 including the objects 40 rotated at random angles around predetermined positions in the objects 40 with respect to the preset orientation of the objects 40. The predetermined position in each object 40 is, for example, a center position or a barycentric position of each object 40. However, the display control unit 23 can also display each object 40 by rotating each object 40 by a preset angle. As illustrated in this example, the test screen 30 displayed by the display control unit 23 can include a mental rotation task.

In one example of the test screen 30, the target object 41 is a simple person illustration of one pattern having one feature, and the non-target object 42 is simple person illustrations of a plurality of patterns having another feature different from the one feature. In this case, the feature of the person illustration is at least one of the body shape, the hairstyle, the presence or absence of the hat, the posture, and the cloth, and the target object 41 and the non-target object 42 are different in at least one of these. In one example, when the display control unit 23 displays a plurality of target objects 41 of one pattern having the same feature, the display control unit 23 displays each of the plurality of target objects 41 rotated by a random angle or a preset angle. In this case, the display control unit 23 display, by rotating at a random angle or a preset angle, each of one or a plurality of non-target objects 42 of each pattern of the non-target objects 42 of a plurality of patterns each having the same feature.

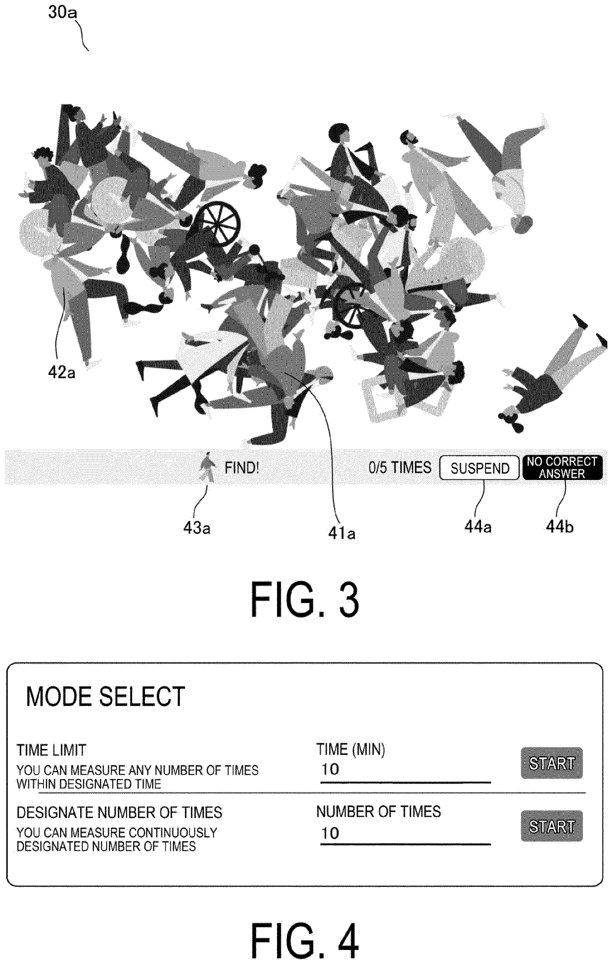

FIG. 3 is a view illustrating an example (test screen 30a) of the test screen 30 displayed by the display control unit 23. The display control unit 23 displays the test screen 30a including a plurality of target objects 41a and a plurality of non-target objects 42a. The test screen 30a includes a reference object 43a indicating the target object 41a to be selected by the test subject. The test screen 30a includes a UI object 44a to be selected in a case where the test screen 30a is suspended, and a UI object 44b to be selected in a case where there is no target object same as the reference object 43a. The test screen 30a includes the target object 41a and the non-target object 42a each arranged in a state of being rotated by a random angle. The test screen 30a displayed in FIG. 3 is a mental rotation task.

When the display control unit 23 displays the test screen 30, the input processing unit 24 determines whether or not the target object 41 is selected on the basis of the input of the test subject received by the input unit 21.

The input processing unit 24 determines whether or not the target object 41 or the non-target object 42 is selected on the basis of the input of the test subject received by the input unit 21. This determination processing allows the input processing unit 24 to also determine whether or not the input of the test subject received by the input unit 21 is an input corresponding to selection of the target object 41 or the non-target object 42. When the input of the test subject received by the input unit 21 is an input corresponding to selection of the target object 41 or the non-target object 42, the input processing unit 24 receives the input of the test subject as an object selection input.

In the present embodiment, when the target object 41 is selected, the input processing unit 24 stores information indicating the target object 41 and the clock time at which the input unit 21 receives the input corresponding to the selection in association with each other in a predetermined memory area in the storage device 15. When the non-target object 42 is selected, the input processing unit 24 stores information indicating the non-target object 42 and the clock time at which the input unit 21 receives the input corresponding to the selection in association with each other in a predetermined memory area in the storage device 15. In the present embodiment, the control unit 22 stores, in a predetermined memory area in the storage device 15, the clock time at which the display control unit 23 starts to display the test screen 30.

In one example, the input processing unit 24 compares the touch position acquired from the input unit 21 with the positions of the target object 41 and the non-target object 42. In a case where the acquired touch position is within a range of the target object 41, the input processing unit 24 determines that the target object 41 is selected, and receives the input as a selection input of the target object 41. In a case where the acquired touch position is within a range of the non-target object 42, the input processing unit 24 determines that the non-target object 42 is selected, and receives the input as a selection input of the non-target object 42. When the acquired touch position is not within the range of the target object 41 or the non-target object 42, the input processing unit 24 does not receive, as a selection input, the input of the test subject received by the input unit 21.

In the present embodiment, while a predetermined condition is satisfied, the display control unit 23 sequentially displays different test screens 30 on the display device 13, and the input processing unit 24 determines whether or not the target object 41 is selected on the basis of the input of the test subject received by the input unit 21. The predetermined condition is a predetermined condition for the test performed by the evaluation device 1, and is, for example, being within a predetermined time after the display control unit 23 displays the test screen 30, or the number of test screens 30 sequentially displayed by the display control unit 23 being within a predetermined number.

The evaluation unit 25 evaluates the temporal lobe function related to the visual recognition ability of the test subject on the basis of the response time required for the test subject to input when the input processing unit 24 determines that the target object 41 is selected. In the present embodiment, the evaluation unit 25 evaluates the temporal lobe function by determining the index of the temporal lobe function related to the visual recognition ability of the test subject. For example, the value of the index of the temporal lobe function indicates that the higher the value is, the more the temporal lobe is activated or used, the shorter the response time is, the higher the value is determined, and the longer the response time is, the lower the value is determined.

In one example, when the input processing unit 24 determines that the target object 41 is selected, the evaluation unit 25 determines, as the response time, the time from when the display control unit 23 displays the test screen 30 until the input processing unit 24 receives an object selection input or the time from when the input processing unit 24 receives an object selection input immediately before it receives the object selection input until it receives the object selection input, and determines the index of the temporal lobe function on the basis of the determined response time.

In one example, in a case where the display control unit 23 displays, on the display device 13, the test screen 30 including the plurality of target objects 41, the evaluation unit 25 determines the response time in each case where the input processing unit 24 determines that the target object 41 is selected, and determines the index of the temporal lobe function on the basis of the determined response time. In this case, for example, the evaluation unit 25 determines the index of the temporal lobe function by calculating the average (average response time) of the response times determined in each case where the input processing unit 24 determines that the target object 41 is selected.

In one example, the evaluation unit 25 evaluates the temporal lobe function related to the visual recognition ability of the test subject by determining a response time on each of the test screens 30, and calculating, for example, an average (average response time) of the determined response times on the basis of the determined response time. For example, the evaluation unit 25 makes a determination by calculating the index of the temporal lobe function by using a correspondence table or a correspondence relationship between a predetermined response time and the index of the temporal lobe function for the test performed on the test subject by the evaluation device 1. For example, the test performed on the test subject by the evaluation device 1 is set in a plurality of patterns, and the evaluation device 1 displays a set of different test screens 30 for each pattern. In this case, a plurality of persons are caused to take tests of a plurality of patterns presented by the evaluation device 1 in advance, an average response time is determined for each test pattern displayed by the evaluation device 1, and the storage device 15 stores the determined average response time as sample data for each person. The evaluation unit 25 can determine the temporal lobe function related to the visual recognition ability of the test subject, and determine relative evaluation by comparing with sample data.

In another example, the evaluation unit 25 evaluates the temporal lobe function related to the visual recognition ability of the test subject by determining an index of the temporal lobe function on each test screen 30, and calculating, for example, an average (average index) of the determined index on the basis of the determined index. For example, the evaluation unit 25 makes a determination by calculating the index of the temporal lobe function by using a correspondence table or a correspondence relationship between a predetermined response time and the index of the temporal lobe function for each test screen 30 displayed by the evaluation device 1. In this case, a plurality of persons are caused to take a test presented by the evaluation device 1 in advance, an average index is determined for each test screen 30 displayed by the evaluation device 1, and the storage device 15 stores the determined average index as sample data for each person. The evaluation unit 25 can determine the temporal lobe function related to the visual recognition ability of the test subject, and determine relative evaluation by comparing with sample data.

In the present embodiment, when the ratio between the number of times the target object 41 is determined to have been selected by the input processing unit 24 and the number of times that the non-target object 42 is determined to have been selected is within a predetermined range, the evaluation unit 25 outputs information indicative of not evaluating the temporal lobe function or not able to evaluate the temporal lobe function. The ratio between the number of times the target object 41 is determined to have been selected and the number of times the non-target object 42 is determined to have been selected corresponds to the correct answer rate of selecting the target object 41 that should be selected.

The visual line detection unit 26 detects visual line information of the test subject on the basis of, for example, corneal reflection of and the pupil position of the test subject by analyzing imaging information of the test subject by the imaging device included in the visual line detection device 14. In one example, the visual line detection unit 26 stores, in a predetermined memory area in the storage device 15, the visual line information of the test subject having been detected, and passes it to the input processing unit 24. In the present embodiment, the input processing unit 24 does not determine that the target object is selected in a case where there is no visual line detected by the visual line detection unit 26 within a predetermined region including the object in the test screen 30 displayed by the display control unit 23.

In the present embodiment, when an evaluation program stored in the storage device 15 is executed, the evaluation device 1 (the control unit 22) starts a test for the test subject via the test screen 30. In one example, the display control unit 23 displays a test mode selection screen 31 and a test start screen 32 before the start of the test, and displays an evaluation screen 33 after the end of the test.

FIG. 4 is a view illustrating an example of the mode selection screen 31 displayed by the display control unit 23. The mode selection screen 31 is a screen for setting a time limit or a designated number of times as a predetermined condition for the test performed by the evaluation device 1. The user of the evaluation device 1 can determine the mode of the test, for example, by inputting a numerical value of the time (minutes) or the number of times, and pressing a "start" button of the time or the number of times. When the display control unit 23 displays the mode selection screen 31, the input processing unit 24 determines a test mode on the basis of the input of the test subject received by the input unit 21. When the test mode is determined, the display control unit 23 displays the start screen 32.

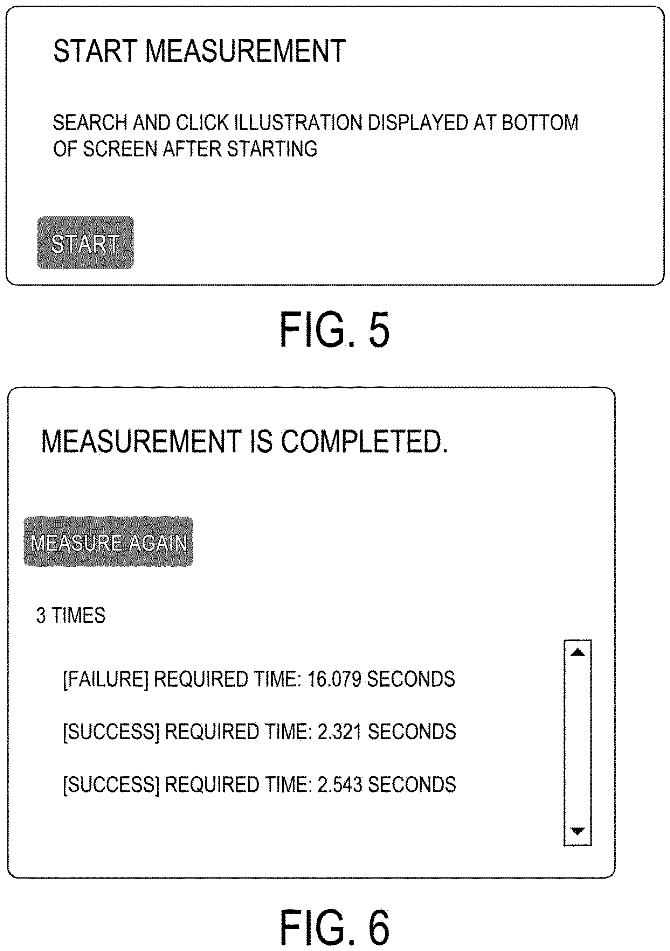

FIG. 5 is a view illustrating an example of the start screen 32 displayed by the display control unit 23. When the display control unit 23 displays the start screen 32, the input processing unit 24 determines starting of the test in a case where the input of the test subject received by the input unit 21 is an input corresponding to "start". When the start of the test is determined, the display control unit 23 displays the test screen 30.

FIG. 6 is a view illustrating an example of the evaluation screen 33 displayed by the display control unit 23. The evaluation screen 33 illustrated in FIG. 6 presents information indicating whether or not the response of the test subject is correct (that is, whether or not to be the target object 41) and response information including the response time required for the test subject to input the response. The evaluation screen 33 illustrated in FIG. 6 indicates that the first selection is the non-target object 42 and the response time for it is 16.079 seconds, the second selection is the target object 41 and the response time for it is 2.321 seconds, and the third selection is the target object 41 and the response time for it is 2.543 seconds. The evaluation screen 33 includes the index of the temporal lobe function related to the visual recognition ability of the test subject (not illustrated).

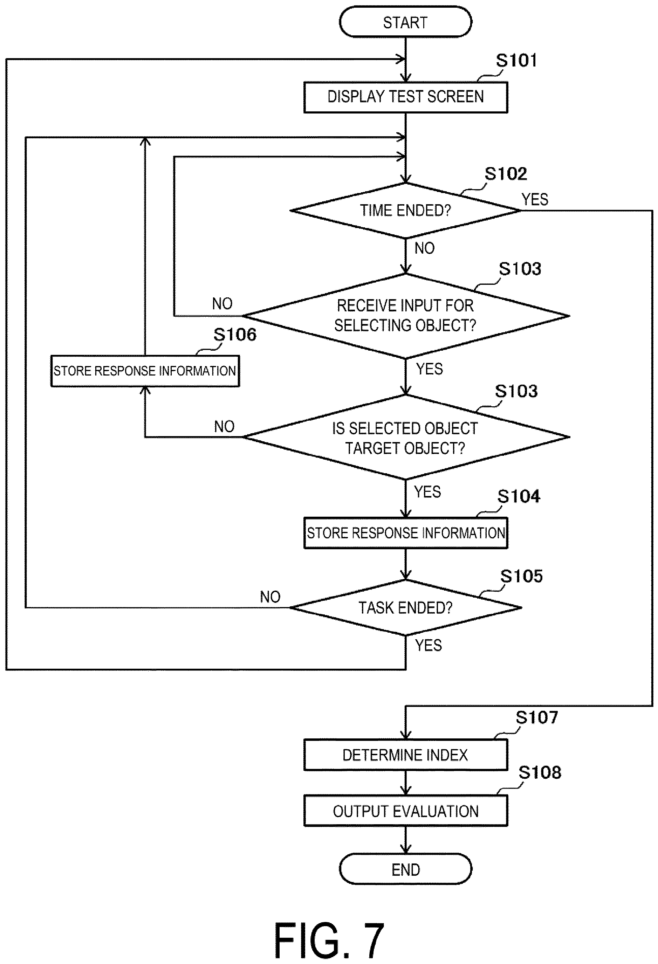

FIG. 7 is a view illustrating an example of a flowchart explaining information processing executed in the evaluation device 1 of one embodiment of the present invention. The present flowchart is a flowchart in a case where the time limit is set as a predetermined condition for the test performed by the evaluation device 1.

In step 101, the display control unit 23 displays the test screen 30. In step 102, the control unit 22 determines whether or not the current clock time is within the time limit set from the clock time when step 101 is first executed, and the present flowchart proceeds to step 103 as long as it is within the time limit.

In step 103, the input processing unit 24 determines whether or not the target object 41 or the non-target object 42 is selected on the basis of the input of the test subject received by the input unit 21. In a case where the target object 41 or the non-target object 42 is not selected, the present flowchart proceeds to step 102. In a case where the target object 41 is selected, the present flowchart proceeds to step 104, and in a case where the non-target object 42 is selected, the present flowchart proceeds to step 106.

In step 104, the input processing unit 24 stores the information indicating the target object 41 and the clock time at which the input unit 21 receives the input corresponding to the selection in association with each other in a predetermined memory area in the storage device 15. In step 106, the input processing unit 24 stores the information indicating the non-target object 42 and the clock time at which the input unit 21 receives the input corresponding to the selection in association with each other in a predetermined memory area in the storage device 15.

In step 105, the control unit 22 determines whether or not to end the test screen 30 being displayed. For example, the control unit 22 determines whether or not the selection of all the target objects 41 included in the test screen 30 being displayed has been completed, and if determining that it has been completed, determines to end the test screen 30. In a case where the control unit 22 determines to end the test screen 30, the present flowchart proceeds to step 101, and in step 101, the display control unit 23 displays a new test screen 30 set in advance. If the control unit 22 determines not to end the test screen 30, the present flowchart proceeds to step 102.

In step 107, the evaluation unit 25 determines the index of the temporal lobe function related to the visual recognition ability of the test subject on the basis of the response time required for the test subject to input when the input processing unit 24 determines that the target object 41 is selected. For example, when the response of the test subject is the response information as illustrated in Fig. 6, since the second and third selections are the target object 41, the evaluation unit 25 determines the index of the temporal lobe function related to the visual recognition ability of the test subject on the basis of the second and third response times. The determination of the index by the evaluation unit 25 in this case is not on the basis of the first response time.

In step 108, the display control unit 23 displays, on the display device 13, the evaluation screen 33 including the index determined in step 107. Alternatively, the control unit 22 transmits data related to the evaluation screen 33 to another device via the communication device 16.

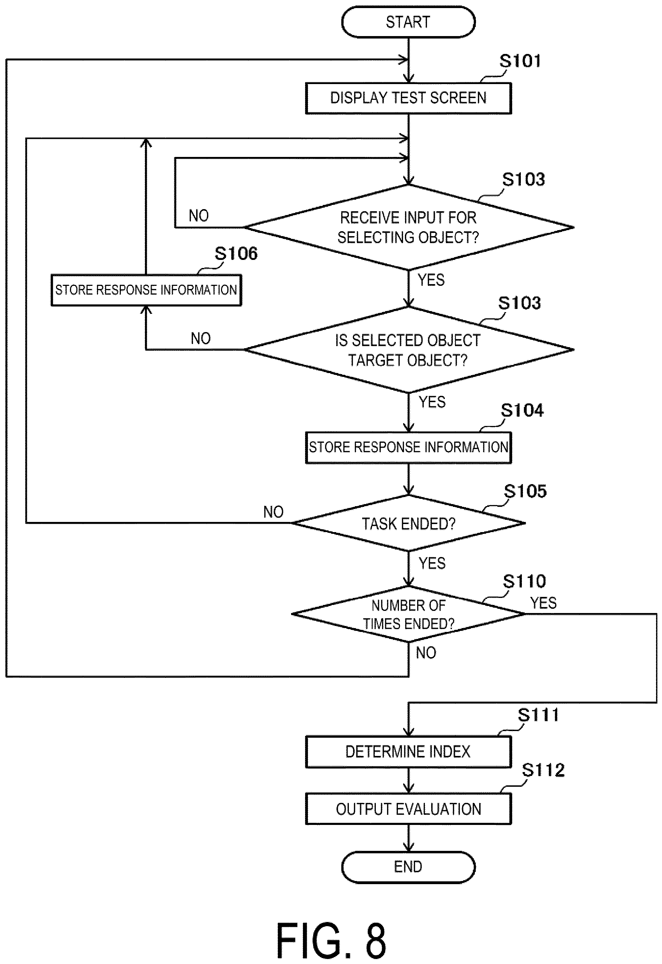

FIG. 8 is a view illustrating an example of a flowchart explaining information processing executed in the evaluation device 1 of one embodiment of the present invention. The present flowchart is a flowchart in a case where the designated number of times is set as a predetermined condition for the test performed by the evaluation device 1. Hereinafter, differences from the flowchart illustrated in FIG. 7 will be mainly described.

The present flowchart includes step 110 in place of including step 102. In step 110, the control unit 22 determines whether or not the number of test screens 30 displayed by the display control unit 23 is the same as the designated number of times that is set. In a case where the designated number of times is determined to be the same, the present flowchart proceeds to step 111, and in a case where the designated number of times is not determined to be the same, that is, in a case where the number of test screens 30 displayed by the display control unit 23 is smaller than the designated number of times that is set, the present flowchart proceeds to step 101. Step 111 and step 112 are the same as step 107 and step 108, respectively.

Next, operations and effects of the evaluation device 1 according to the embodiment of the present invention will be described.

It is known that that various human abilities, advantages, disadvantages, ideas, and the like are caused by a manner of information processing in the brain, that is, a difference in sites and cooperation among sites in the brain that performs information processing. It is known that for example, when humans recognize objects such as an object or a person, there are those whose parietal lobe is mainly activated and those whose temporal lobe is mainly activated. In this regard, for example, Non-Patent Document 1 discloses that there is a difference in response time in a mental rotation task between test subjects whose temporal lobes are activated and other test subjects. The inventors of the present application consider that enabling objective evaluation through quantification of the temporal lobe function when visually recognizing an object, a person, or the like, makes it possible to more appropriately grasp (evaluate) the personalities of individuals from the aspect of brain function. Such technical ideas and technical content have not been considered so far. As a related technique, for example, Patent Document 1 discloses a rehabilitation device for high function disorder of brain that presents a task data to a patient and provides predetermined teaching data according to a state of errors in the response of the patient, but does not disclose a technical idea or technical content related to the evaluation device 1 of the present embodiment.

Although it has not been considered so far to objectively evaluate the temporal lobe function by quantitatively measuring the same in this manner, it has been possible to quantitatively measure the temporal lobe function by using a device such as electroencephalogram or fMRI. However, these devices are large in size, and have a large burden on the test subject because the measurement takes a long time and physical restraint is also involved.

In the present embodiment, the display control unit 23 displays, on the display device 13, the test screen 30 including the target object 41 that the test subject should select and the non-target object 42 that the test subject should not select. The display control unit 23 can display, on the display device 13, the test screen 30 including the non-target object 42 having a predetermined similarity to the target object 41 in shape, color, or pattern. The input processing unit 24 determines whether or not the target object 41 is selected on the basis of the input of the test subject received by the input unit 21. While the predetermined condition is satisfied, the display control unit 23 sequentially displays different test screens 30 on the display device 13, and the input processing unit 24 determines whether or not the target object 41 is selected on the basis of the input of the test subject received by the input unit 21. The evaluation unit 25 evaluates the temporal lobe function related to the visual recognition ability of the test subject on the basis of the response time required for the test subject to input when the input processing unit 24 determines that the target object 41 is selected.

As described above, by causing the test subject to select the target object 41 from the plurality of objects 40 that are possibly give the test subject a similar impression when viewed at a glance and determining the response time when the test subject selects the target object 41 (when giving a correct answer), the present embodiment makes it possible to objectively evaluate the temporal lobe function when visually recognizing an object, a person, and the like while further reducing the burden on the test subject. This makes it possible to more appropriately grasp the personality of individuals from the aspect of brain function.

In the present embodiment, when the ratio between the number of times the target object 41 is determined to have been selected by the input processing unit 24 and the number of times that the non-target object 42 is determined to have been selected is within a predetermined range, the evaluation unit 25 outputs information indicative of not evaluating the temporal lobe function or not able to evaluate the temporal lobe function. With such a configuration, in the present embodiment, the evaluation device 1 becomes able to perform evaluation only in a case of equal to or higher than a predetermined correct answer rate. This makes it possible to evaluate only the test subject who properly checks the test screen 30, and possible to more appropriately evaluate the temporal lobe function related to the visual recognition ability of the test subject.

For example, the evaluation unit 25 can be configured to evaluate or not to evaluate the temporal lobe function for each test screen 30 according to the correct answer rate. This makes it possible to evaluate only the test screen 30 on which response to the test is intensive, and it is possible to more appropriately evaluate the temporal lobe function related to the visual recognition ability of the test subject. For example, it is possible to for the evaluation device 1 to perform evaluation only in a case where all answers are correct, and in this case, it is possible to evaluate only a case where the test is intensively and appropriately answered, making it possible to more appropriately evaluate the temporal lobe function related to the visual recognition ability of the test subject.

In the present embodiment, the input processing unit 24 does not determine that the target object 41 is selected in a case where there is no visual line detected within the predetermined region including the object 40 in the test screen 30 displayed by the display control unit 23. Such a configuration allows the present embodiment to perform evaluation only in a case where the target object 41 is selected while checking the test screen 30. This makes it possible to evaluate only the test subject who properly checks the test screen 30, and possible to more appropriately evaluate the temporal lobe function related to the visual recognition ability of the test subject.

The inventors of the present application have proposed, in Japanese Patent Application No. 2020-082634, an evaluation system for quantifying the function of the parietal lobe when visually recognizing an object, a person, or the like. Combined use of the evaluation device 1 of the embodiment of the present invention and the evaluation system makes it possible to objectively evaluate, through quantification, the parietal lobe and temporal lobe functions when visually recognizing an object, a person, and the like, and possible to more appropriately grasp personalities of individuals from the aspect of brain function.

The above operations and effects are the same in other embodiments and other examples unless otherwise specified.

In another embodiment of the present invention, a program for implementing the functions of the embodiment of the present invention described above and the information processing illustrated in the flowcharts and a computer-readable storage medium storing the program can be provided. In yet another embodiment, a method for implementing the functions of the embodiment of the present invention described above and the information processing illustrated in the flowcharts can be provided. In yet another embodiment, a server that can supply, to a computer, a program for implementing the functions of the embodiment of the present invention described above and the information processing illustrated in the flowcharts can be provided. In still another embodiment, a virtual machine that implements the functions of the embodiment of the present invention described above and the information processing illustrated in the flowcharts can be provided.

In one embodiment of the present invention, the input device 12 can be not the touchscreen 18 but a mouse, and the display device 13 can be a display that is not the touchscreen 18. In this case, clicking of a pointer of the mouse corresponds to touch input to the touchscreen 18.

In one embodiment of the present invention, the display device 13 can be not the touchscreen 18 but a head mounted display (HMD). For example, the HMD is configured such that an electronic display is built in a housing having a goggles-like shape, and an image is displayed in a visual line direction of the user wearing the HMD. In this case, the input device 12 can be any device, sensor, or the like associated with the HMD.

In one embodiment of the present invention, the evaluation unit 25 can also be configured to evaluate the temporal lobe function regardless of the ratio between the number of times the target object 41 is determined to have been selected by the input processing unit 24 and the number of times that the non-target object 42 is determined to have been selected.

In one embodiment of the present invention, the evaluation device 1 can include none of the visual line detection device 14. In this case, the evaluation device 1 does not include the visual line detection unit 26 as a functional block.

In one embodiment of the present invention, when displaying the test screen 30 to present a mental rotation task, the display control unit 23 displays the test screen 30 including the object 40 (the target object 41 and the non-target object 42) having more complex stimulating elements (color, shape, and pattern) to certain extent or more. For example, the object 40 can be a stereoscopic figure or the like having more complex stimulating elements (color, shape, and pattern) to certain extent or more. It is reported that when a figure or the like presented in a mental rotation task has a certain difficulty level, object recognition of the temporal lobe system is enhanced during execution of the task even without performing the mental rotation (Non-Patent Document 2). Therefore, if the temporal lobe system is enhanced while measuring the visual recognition characteristics through a mental rotation task, there is a case of failing to accurately measure the visual recognition characteristics. With the configuration in which the display control unit 23 displays the test screen 30 including the object 40 having more complex stimulating elements to some extent or more when presenting a mental rotation task, even when the test is performed a plurality of times for the test subject, it is possible to more appropriately evaluate the temporal lobe function related to the visual recognition ability of the test subject.

In one example of the test screen 30 of the embodiment of the present invention, the non-target object 42 is a person image of one person, and the target object 41 is a face image is the person image of the one person subjected to predetermined processing. For example, in this case, the target object 41 is a face image subjected to only processing of replacing the face part of one person of the non-target object 42 with the face part of another person.

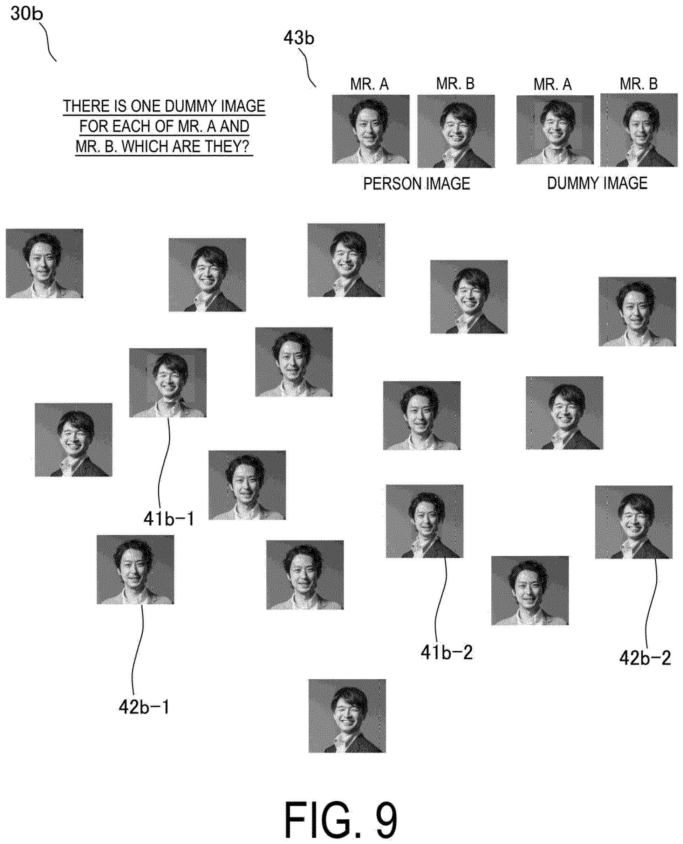

FIG. 9 is a view illustrating an example (test screen 30b) of the test screen 30 displayed by the display control unit 23. The display control unit 23 displays the test screen 30b including target objects 41b-1 and 41b-2 and non-target objects 42b-1 and 42b-2. The non-target object 42b-1 is a normal image of Mr. A, and the target object 41b-1 is a dummy image in which a main part (part in the face including eyes, nose, mouth, and eyebrows) of the face of the person image of Mr. A is replaced with a corresponding part of Mr. B. The non-target object 42b-2 is a normal image of Mr. B, and the target object 41b-2 is a dummy image in which a main part (part in the face including eyes, nose, mouth, and eyebrows) of the face of the person image of Mr. B is replaced with a corresponding part of Mr. A.

The test screen 30b is a screen for displaying the plurality of normal images of Mr. A and the plurality of normal images of Mr. B, displaying one dummy image of the person image of Mr. A and one dummy image of the person image of Mr. B, and causing the test subject to select the dummy image. The test screen 30b displays a relatively large number of, for example, 10 or more of normal images of Mr. A and normal images of Mr. B. The test screen 30b can include an area for displaying reference objects 43b of normal images and dummy images of Mr. A and Mr. B.

In one example of the test screen 30 of the embodiment of the present invention, the target object 41 is an illustration of a palm of the right hand, and the non-target object 42 is an illustration of a palm of the left hand. For example, in this case, each of the target object 41 and the non-target object 42 is displayed in a state of being rotated by a random angle.

In one example of the test screen 30 of the embodiment of the present invention, there is one target object 41, and the one target object 41 is the UI object 44 selectable by the test subject including a message for the test subject. In this case, the display control unit 23 displays, on the display device 13, the test screen 30 including only the one target object 41. Also in this case, the evaluation unit 25 evaluates the temporal lobe function related to the visual recognition ability of the test subject on the basis of the response time required for the test subject to input when the input processing unit 24 determines that the target object 41 is selected.

Hereinafter, modifications of the embodiment of the present invention will be described. The modifications described below can be appropriately combined and applied to any embodiment of the present invention as long as no contradiction arises.

In one modification, the evaluation unit 25 evaluates the temporal lobe function related to the visual recognition ability of the test subject on the basis of a ratio (γ) between the number of times (α) that the target object 41 is determined to have been selected by the input processing unit 24 and the number of times (β) that the non-target object 42 is determined to have been selected, and a response time (δ) required for the test subject to input when the input processing unit 24 determines that the target object 41 is selected. For example, the evaluation unit 25 calculates the ratio (γ) corresponding to the correct answer rate (α/(α + β)), calculates the average response time (δ), and calculates the index of the temporal lobe function by the ratio (y)/the average response time (δ).

In one modification, the display control unit 23 displays only one test screen 30 on the display device 13. On the test screen 30, the evaluation unit 25 evaluates the temporal lobe function related to the visual recognition ability of the test subject on the basis of the response time required for the test subject to input when the input processing unit 24 determines that the target object 41 is selected.

In the processing or operations described above, the processing or operations can be modified freely as long as there is no occurrence of contradiction in the processing or operations such as utilization at a certain step of data that still cannot be utilized at that step. Additionally, each example described above is exemplified for describing the present invention, and the present invention is not limited to these examples. The present invention may be implemented in various forms without departing from the scope thereof.

### Reference Signs List

1 evaluation device
11 processor
12 input device
13 display device
14 visual line detection device
15 storage device
16 communication device
17 bus
18 touchscreen
21 input unit
22 control unit
30 test screen
31 mode selection screen
32 start screen
33 evaluation screen
40 object
41 target object
42 non-target object
43 reference object
44 UI object

## Claims

1. An evaluation device for evaluating a test subject's ability to identify an object, the evaluation device comprising:
an input unit that receives input of the test subject;
a display control unit that displays, on a display device, a test screen including a target object that the test subject should select and a non-target object that the test subject should not select;
an input processing unit that determines whether or not a target object is selected on a basis of input of the test subject received by the input unit; and
an evaluation unit that evaluates the test subject's ability to identify an object on a basis of a response time required for the test subject to input when the input processing unit determines that the target object is selected.

2. The evaluation device according to claim 1, wherein
when the input of the test subject received by the input unit is an input corresponding to selection of a target object or a non-target object, the input processing unit receives the input of the test subject as an object selection input, and
when the input processing unit determines that the target object is selected, the evaluation unit determines, as a response time, a time from when the display control unit displays a test screen to when the input processing unit receives an object selection input or a time from when the input processing unit receives an object selection input immediately before the object selection input to when the input processing unit receives the object selection input, and determines an index of the ability to identify an object on a basis of the determined response time to evaluate the test subject's ability to identify an object.

3. The evaluation device according to claim 1 or 2, wherein
the display control unit displays, on a display device, a test screen including a plurality of target objects, and
the evaluation unit evaluates the test subject's ability to identify an object by determining a response time in each case where the input processing unit determines that a target object is selected, and determining an index of the ability to identify an object on a basis of the determined response time.

4. The evaluation device according to any one of claims 1 to 3, wherein the display control unit displays a test screen including a non-target object that has a predetermined similarity to the target object in figure feature, color, or pattern target but is different from the target object in figure feature, color, or pattern target.

5. The evaluation device according to any one of claims 1 to 4, wherein the display control unit displays, on the display device, a test screen including a target object and a non-target object rotated at a random angle around a predetermined position in each of the objects.

6. The evaluation device according to any one of claims 1 to 3, wherein the display control unit displays, on the display device, a test screen including only one target object, and the target object is a UI object selectable by a test subject including a message for the test subject.

7. The evaluation device according to any one of claims 1 to 6, wherein
the display control unit sequentially displays different test screens on the display device while satisfying a predetermined condition, and
the evaluation unit evaluates the test subject's ability to identify an object by determining a response time on each of the test screens, and determining an index of the ability to identify an object on a basis of the determined response time.

8. The evaluation device according to any one of claims 1 to 7, wherein
the input processing unit determines whether or not a target object or a non-target object is selected on a basis of an input of the test subject received by the input unit, and
the evaluation unit outputs information indicative of not evaluating the ability to identify an object or not being able to evaluate the ability to identify an object in a case where a ratio between a number of times the input processing unit determines that the target object is selected and a number of times the input processing unit determines that the non-target object is selected falls within a predetermined range.

9. The evaluation device according to any one of claims 1 to 8 further comprising
a visual line detection unit that detects a visual line of the test subject,
wherein the input processing unit does not determine that a target object is selected in a case where the detected visual line is not present in a predetermined region including an object in a test screen displayed by the display control unit.

10. The evaluation device according to any one of claims 1 to 9, wherein the evaluation unit evaluates the test subject's ability to identify an object on a basis of a ratio between a number of times the input processing unit determines that the target object is selected and a number of times the input processing unit determines that the non-target object is selected, and a response time required for the test subject to input when the input processing unit determines that the target object is selected.

11. A method for evaluating a test subject's ability to identify an object, the method comprising:
a step of displaying, on a display device, a test screen including a target object that the test subject should select and a non-target object that the test subject should not select;
a step of determining whether or not a target object is selected on a basis of input of the test subject having been received; and
a step of evaluating the ability to identify an object on a basis of a response time required for the test subject to input when the target object is determined to have been selected.

12. A program for causing a computer to execute each of the steps of the method according to claim 11.
